# EUROPEAN PATENT SPECIFICATION

(11) **EP 2 385 045 B1**
(45) Date of publication and mention of the grant of the patent: **16.09.2015**
(21) Application number: 10735847.5
(22) Date of filing: 21.01.2010
(51) Int. Cl.: C07D 313/12

(54) **PROCESS FOR PRODUCING DIBENZOXEPIN COMPOUND**
VERFAHREN ZUR HERSTELLUNG EINER DIBENZOXEPINVERBINDUNG
PROCÉDÉ DE PRODUCTION D'UN COMPOSÉ DE DIBENZOXÉPINE

(30) Priority: 30.01.2009 JP 2009020292
(43) Date of publication of application: 09.11.2011
(73) Proprietor: Sumitomo Chemical Company, Limited, Tokyo 104-8260 (JP)
(72) Inventor: SASAYAMA, Daisuke, Toyonaka-shi Osaka 561-0802 (JP); HAYASHI, Taketo, Sanda-shi Hyogo 669-1547 (JP)
(74) Representative: Vossius & Partner Patentanwälte Rechtsanwälte mbB
(86) International application number: PCT/JP2010/051070
(87) International publication number: WO 2010/087381

(56) References cited:
- WO-A1-2008/099900
- WO-A1-2008/099900
- WO-A2-2007/105234
- WO-A2-2007/110761
- JP-A- 2 028 124
- JP-A- 9 013 023
- JP-A- 10 226 656
- JP-A- 11 349 524
- JP-A- 2002 302 485
- JP-T- 2001 505 189
- JP-T- 2004 524 316
- OHSHIMA E ET AL: "SYNTHESIS AND ANTIALLERGIC ACTIVITY OF 11-(AMINOALKYLIDENE)-6,11-DIHYDRODIBENZÚB, E 3/4 OXEPIN DERIVATIVES", JOURNAL OF MEDICINAL CHEMISTRY, AMERICAN CHEMICAL SOCIETY, US, vol. 35, no. 11, 1 May 1992 (1992-05-01), pages 2074-2084, XP000615220, ISSN: 0022-2623, DOI: 10.1021/JM00089A020

## Description

### TECHNICAL FIELD

The present invention relates to a process for producing (Z)-11-(3-dimethylaminopropylidene)-6,11-dihydrodibenz[b,e] oxepin-2-acetic acid or an acid addition salt thereof, which is useful as a medicinal agent.

### BACKGROUND ART

(Z)-11-(3-dimethylaminopropylidene)-6,11-dihydrodibenz[b,e]oxepin-2-acetic acid is a compound represented by Formula [II]: 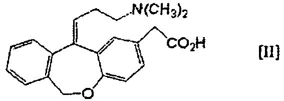 and is a useful pharmaceutical compound to be applied to allergic diseases such as hay fever, allergic rhinitis and urticaria (USP 5,116,863).

In the production of (Z)-11-(3-dimethylaminopropylidene)-6,11-dihydrodibenz[b,e]oxepin-2-acetic acid, generally, an E isomer is produced at the same time, and, therefore, isomerization is necessary to efficiently obtain the Z isomer of interest.

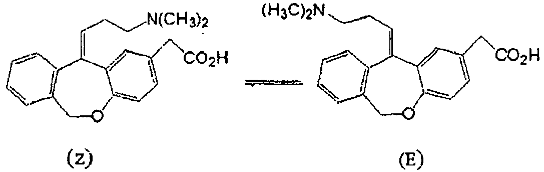

WO 2007/105234 discloses heating 11-hydroxy-11-(3-dimethylaminopropyl)-6,11-dihydrodibenz[b,e]oxepin-2-acetic acid in the presence of hydrochloric acid at 90°C; thereafter, extracting, with methylene chloride, a solution containing a mixture of E and Z isomers of 11-hydroxy-11-(3-dimethylaminopropylidene)-6,11-dihydrodibenz[b,e]oxepin-2-acetic acid; and adding a poor solvent such as acetone to deposit a precipitate, thereby obtaining the Z isomer, the E isomer being isolated by column chromatography for recovery thereof. The yields of the Z and E isomers are 25. 4% and 67%, respectively, and the yield of the Z isomer, olopatadine, is very low. Thus, this process is not suitable for an industrial production process.

WO 2008/099900 discloses that (2)-11-(3-dimethylaminopropylidene)-6,11-dihydrodibenz[b,e]oxepin-2-acetic acid can be efficiently produced by heating an ester of 11-hydroxy-11-(3-dimethylaminopropyl)-6,11-dihydrodibenz [b,e]oxepin-2-acetic acid in the presence of an acid in a solvent.

### DISCLOSURE OF THE INVENTION

An object of the present invention is to provide a process for producing (Z)-11-(3-dimethylaminopropylidene)-6,11-dihydrodibenz[b,e]oxepin-2-acetic acid or an acid addition salt thereof, which is useful as a medicinal agent, in an efficient and industrially advantageous manner.

According to the present invention, 11-hydroxy-11-(3-dimethylaminopropyl)-6,11-dihydrodibenz[b,e]oxepin-2-acetic acid or a salt thereof is subjected to a heat treatment and a water removal treatment in a reaction system in the presence of a specific acid in a solvent, so that the isomerization thereof to a Z isomer proceeds, thereby enabling the production of (Z)-11-(3-dimethylaminopropylidene)-6,11-dihydrodibenz [b,e]oxepin-2-acetic acid or an acid addition salt thereof in an efficient and industrially easy manner.

That is, the present invention is as follows.
[1] A process for producing (Z)-11-(3-dimethylaminopropylidene)-6,11-dihydrodibenz[b,e]oxepin-2-acetic acid represented by Formula [II]: 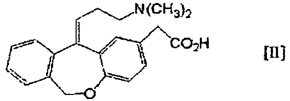 or an acid addition salt thereof, the process including subjecting 11-hydroxy-11-(3-dimethylaminopropyl)-6,11-dihydrodibenz[b,e]oxepin-2-acetic acid represented by Formula [I]: 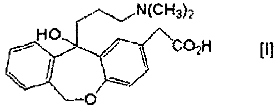 or a salt thereof to a heat treatment in a solvent and a water removal treatment in a reaction system in the presence of an acid selected from a group consisting of hydrogen chloride, sulfuric acid, methanesulfonic acid and p-toluenesulfonic acid.
[2] The process according to [1], wherein the water removal treatment in the reaction system is performed by using a C₃-C₈ carboxylic acid anhydride.
[3] The process according to [2], wherein the C₃-C₈ carboxylic acid anhydride is acetic anhydride.
[4] The process according to [1], wherein the water removal treatment in the reaction system is performed by azeotropic distillation.
[5] The process according to [4], wherein the azeotropic distillation is performed under reduced pressure.
[6] The process according to [1], wherein the water removal treatment in the reaction system is performed by using a C₂-C₈ carboxylic halide.
[7] The process according to [6], wherein the C₂-C₈ carboxylic halide is acetyl chloride.
[8] The process according to [1], wherein the water removal treatment in the reaction system is performed by using an oxyhalide of sulfur or phosphorus.
[9] The process according to [1], wherein the acid is hydrogen chloride.
[10] The process according to [1], wherein the heat treatment is performed at 70 to 120°C.
[11] The process according to [1], which is performed in the presence of a seed crystal of (Z)-11-(3-dimethylaminoprcpylidene)-6,11-dihydrodibenz[b,e]oxepin-2-acetic acid or an acid addition salt thereof.
[12] The process according to [1], wherein the organic solvent is toluene or chlorobenzene.

### DISCLOSURE OF THE INVENTION

The salt of 11-hydroxy-11-(3-dimethylaminopropyl)-6,11-dihydrodibenz[b,e]oxepin-2-acetic acid represented by Formula [I] (Compound [I]) includes acid addition salts such as a hydrochloride, a hydrobromide, a sulfate, a methanesulfonate and a p-toluenesulfonate.

The salt of (Z)-11-(3-dimethylaminopropylidene)-6,11-dihydrodibenz[b,e]oxepin-2-acetic acid represented by Formula [II] (Compound [II]) includes acid addition salts such as a hydrochloride, a hydrobromide, a sulfate, a methanesulfonate and a p-toluenesulfonate.

Compound [II] can be produced by subjecting Compound [I] to a heat treatment and a water removal treatment in the presence of an acid selected from the group consisting of hydrogen chloride, sulfuric acid, methanesulfonic acid and p-toluenesulfonic acid in a solvent.

Hydrogen chloride is a preferable acid. Hydrogen chloride may be in the form of a solution of an organic solvent, but is preferably used in the form of a gas, and, in that case, is usually blown into a reaction system for use.

The amount of the acid to be used is preferably a proportion of usually from 2 to 5 mol, and preferably from 2.5 to 3.5 mol with respect to 1 mol of Compound [I] from the viewpoint of reaction rate and suppression of production of an E isomer and impurities.

An organic solvent is preferably used. The organic solvent includes, for example, ketone solvents (such as methyl ethyl ketone, methyl isobutyl ketone, cyclohexanone and cyclopentanone) and aromatic solvents (such as benzene, toluene, xylene, chlorobenzene, dichlorobenzene and nitrobenzene), and is preferably toluene or chlorobenzene, and particularly preferably toluene.

The amount of the solvent to be used is preferably a proportion of usually from 10 L to 20 L, and preferably from 13 L to 16 L with respect to 1 kg of Compound [I] from the viewpoint of prevention of scaling and economic efficiency.

Specific examples of a reaction method include: 1) a method including mixing a solvent, Compound [I] and an acid, and then subjecting the mixture to a heat treatment and a water removal treatment; 2) a method including adding Compound [I] to a solution containing a mixture of a solvent and an acid while subjecting Compound [I] to a heat treatment, followed by a water removal treatment; and 3) a method including mixing and heating a solvent and an acid, adding dropwise Compound [I] dissolved in the solvent, and then performing a water removal treatment. Also, the heat treatment and the water removal treatment in the reaction system may be performed at the same time.

The chemical reaction of the present invention proceeds in accordance with the following scheme. 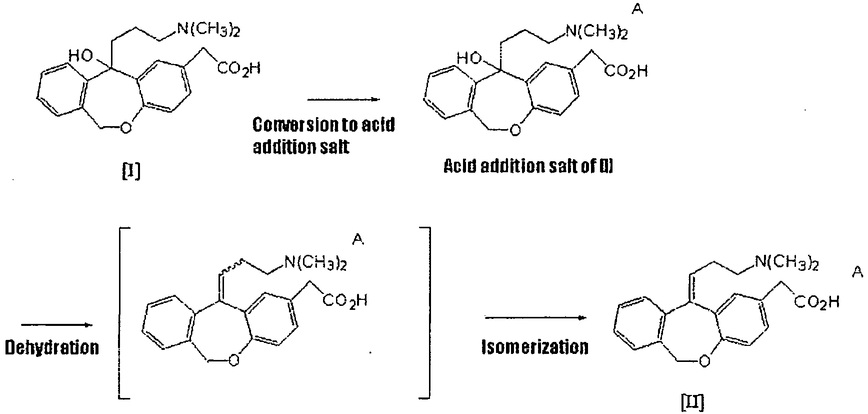 In the scheme, A represents an acid.

First, Compound [I] is converted, by an acid, into the corresponding acid addition salt. Dehydration (propylidenation at 11 position) proceeds due to heating, whereby water is generated. This generated water is removed from the reaction system so that dehydration is promoted and isomerization of Compound [I] to a Z isomer proceeds.

The heat treatment is performed usually at 50°C to 150°C, preferably at 70°C to 120°C, and more preferably at 90°C to 110°C from the viewpoint of reaction rate and suppression of production of impurities.

Examples of the method for water removal treatment in the reaction system include a method adding, for example, a C₃-C₈ carboxylic acid anhydride, a C₂-C₈ carboxylic halide or an oxyhalide of sulfur or phosphorus; a method by azeotropic distillation; and the like.

C₃-C₈ carboxylic acid anhydride means a carboxylic acid anhydride wherein the number of carbon atoms in the entire acid anhydride ranges from C₃ to C₈, and includes, for example, acetic anhydride, propionic anhydride, butyric anhydride, formic-acetic mixed acid anhydride, acetic-propionic mixed acid anhydride, succinic anhydride and maleic anhydride, and is preferably acetic anhydride.

The amount of C₃-C₈ carboxylic acid anhydride to be used is a proportion of usually from 1 mol to 2 mol, and preferably from 1 to 1.5 mol with respect to 1 mol of Compound [I] from the viewpoint of prevention of scaling and economic efficiency.

C₂-C₈ carboxylic halide includes, for example, C₂-C₈ carboxylic chlorides such as acetyl chloride, propionyl chloride, oxalyl chloride and benzoyl chloride; C₂-C₈ carboxylic bromides such as acetyl bromide, propionyl bromide, oxalyl bromide and benzoyl bromide, and is preferably a C₂-C₈ carboxylic chloride, particularly acetyl chloride.

The amount of C₂-C₈ carboxylic halide to be used is a proportion of usually from 1 mol to 2 mol, and preferably from 1 to 1.5 mol with respect to 1 mol of Compound [I] from the viewpoint of prevention of scaling and economic efficiency.

Examples of the oxyhalide of sulfur or phosphorus include thionyl chloride and phosphorus oxychloride.

The amount of the oxyhalide of sulfur or phosphorus to be used is a proportion of usually from 0.5 mol to 2 mol, and preferably from 1 to 1.5 mol with respect to 1 mol of Compound [I] from the viewpoint of prevention of scaling and economic efficiency.

The azeotropic distillation can be performed, for example, by a method using a Dean-Stark apparatus. Additionally, the azeotropic distillation is preferably performed under reduced pressure. When the azeotropic distillation is employed, it is necessary to select an organic solvent which can cause azeotropy with water.

The order of azeotropic distillation includes, for example, 1) a method of performing azeotropic distillation simultaneously with a heat treatment and 2) a method of performing azeotropic distillation after a heat treatment. However, it is preferred, in the present invention, that azeotropic distillation be performed after a heat treatment, followed by an additional heat treatment.

A preferred water removal treatment method is a method for water removal treatment by adding a C₃-C₈ carboxylic acid anhydride, by adding a C₂-C₈ carboxylic halide, or by azeotropic distillation.

The reaction time usually ranges from 1 hour to 12 hours, and preferably 1 hour to 6 hours, depending on the reaction temperature, amounts of raw materials to be used and the like. This reaction is preferably carried out under stirring. While the reaction time depends on the reaction temperature, amounts of raw materials to be used and the like, the reaction is preferably carried out under stirring.

The reaction may be carried out in the presence of a seed crystal of Compound [II]. Due to this, the reaction easily proceeds, thereby enabling the suppression of scaling to a reaction container and the reduction in dropping time. The amount of the seed crystal to be used is a proportion of preferably from 0.1 to 2 parts by weight, and more preferably from 0.5 to 1.5 parts by weight with respect to 100 parts by weight of Compound [I]. In the meantime, the seed crystal is preferably added into the system during feeding of raw materials from the viewpoint of operability.

The raw material, Compound [I], to be used in the production process according to the present invention can be produced by reacting 11-oxo-6,11-dihydrodibenz[b,e]oxepin-2-acetic acid represented by Formula [III] (Compound [III]) and a Grignard reagent represented by Formula [IV] in accordance with the method described in WO 2008/099900. 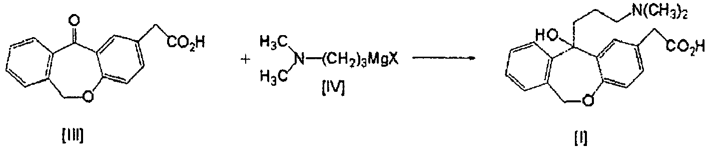 In the formula, X represents a chlorine atom or a bromine atom.

Additionally, Compound [III] can be produced by the method described in J. Med. Chem., 19, 941 (1976) or 20, 1499 (1977), or USP 4,282,365.

In this way, dehydration of Compound [I] and isomerization thereof to a Z isomer proceed. While a small quantity of E isomer and Compound [II] as a Z isomer deposited as crystals are present in the reaction system wherein the reaction has almost proceeded, Compound [II] can be acquired as an acid addition salt of the acid used in the reaction, by cooling (preferably, gradually cooling) the reaction mixture to room temperature, thereafter, filtering the reaction mixture to separate crystals therefrom, and then washing the crystals with an appropriate solvent such as acetone.

While Compound [II] is obtained as an acid addition salt through the above reaction, the acid addition salt can be led to free Compound [II] by an alkaline treatment which is a conventional method.

The thus-obtained Compound [II] is a high-purity crystal having a Z isomer/E isomer ratio (area ratio by HPLC measurement) of usually 98/2 or higher, and a high-purity crystal having the ratio of 99/1 or higher can also be obtained.

### Examples

Hereinafter, the present invention will be described by way of Examples, but is not limited thereto. The ratio between an E isomer and a Z isomer of the compound produced was obtained from values measured by high-performance liquid chromatography (HPLC).

### Production Example

### Production of 11-hydroxy-11- (3-dimethylaminopropyl)-6, 11-dihydrodibenz[b,e]oxepin-2-acetic acid

To a tetrahydrofuran (47 mL) solution in which 10 g (37.3 mmol) of 11-oxo-6,11-dihydrodibenz[b,e]oxepin-2-acetic acid was dissolved, 160 mL (corresponding to 117 mmol) of a THF/toluene solution of 3-dimethylaminopropyl magnesium chloride was added dropwise at 5 to 18°C for 65 minutes. THF (94 mL) was added during dropping. The solution was stirred at 14 to 18°C for 1 hour, and acetic acid (15 mL) was added dropwise to the reaction liquid. Further, water (40 mL) was poured into the reaction liquid. The reaction liquid was stood still to separate an organic layer therefrom. An aqueous layer (lower layer) was stood still over one night so that crystals were deposited. The crystals were filtered, washed with cool water (30 mL) having a temperature of about 5°C, and dried under reduced pressure to obtain 10.56 g of a title compound. The yield of the compound was 79.7%.
Melting Point: 203.5°C
¹H NMR (400 MHz, CDCl₃) δ 1.51-1.60 (m, 2H), 2.07-2.11 (m, 1H), 2.49-2.53 (m, 1H), 2.72 (s, 6H), 2.81-2.88 (m, 1H), 2.93-2.99 (m, 1H), 3.50 (s, 2H), 3.73-3.77 (m, 1H), 5.04 (d, J= 15.6 Hz, 1H), 5.46 (d, J=15.6Hz, 1H), 7.09 (d, J=8.0Hz, 2H), 7.24-7.38 (m, 3H), 7.61 (d, J= 2.0 Hz, 1H), 7.89 (dd, J= 8.0, 0.8 Hz, 1H)
(HPLC conditions)
Column: CAPCELL PACK C18 MGIII (4.6 mmi.d. x 15 cm, 5 µm) Mobile phase A: 0.1% trifluoroacetic acid water
Mobile phase B: Methanol/acetonitrile = 1/1
A/B = 8/2 → 1/9 (30 minutes)
Flow rate: 1.0 mL/min.
Column temperature: 30°C
Wavelength detected: UV 254 nm

### Example 1

### (Z)-11-(3-dimethylaminopropylidene)-6,11-dihydrodibenz[b,e] oxepin-2-acetic acid • hydrochloride

To a pressure-resistant container equipped with a 100-mL teflon inner cylinder, 25.37 g of a toluene solution in which 3 g (8.44 mmol) of 11-hydroxy-11-(3-dimethylaminopropyl)-6,11-dihydrodibenz[b,e]oxepin-2-acetic acid, 0.95 g (9.3 mmol) of acetic anhydride and 0.62 g (16.9 mmol) of hydrogen chloride were dissolved was added, and then the container was hermetically sealed. This container was heated up to 100°C, and the solution was stirred for 6 hours. The container was cooled to room temperature, and, thereafter, crystals were filtered, washed with 15 mL of acetone, and then dried under reduced pressure to obtain 2.51 g of (Z)-11-(3-dimethylaminopropylidene)-6,11-dihydrodibenz[b,e]oxepin-2-acetic acid • hydrochloride. The yield was 79.5%. When measured by HPLC, the proportions of the Z isomer and the E isomer were 99.40% and 0.60%, respectively.

### Example 2

### (Z)-11-(3-dimethylaminopropylidene)-6,11-dihydrodibenz[b,e] oxepin-2-acetic acid • hydrochloride

To a flask, 2 g (5.63 mmol) of 11-hydroxy-11-(3-dimethylaminopropyl)-6,11-dihydrodibenz[b,e]oxepin-2-acetic acid and 25.37 g (11.26 mmol) of a 2.42% hydrogen chloride-toluene solution were added, and the mixture was stirred at 90°C for 7 hours. The E isomer/Z isomer ratio of the reaction mixture, when measured by HPLC, was 61.28 : 38.82. Toluene (8 mL) was distilled off at an inner pressure of 430 to 500 hPa and a temperature of 90 to 95°C, and then water was distilled off. The concentrated viscous reaction mixture was transferred to a pressure-resistant container equipped with a 100-mL teflon inner cylinder, and 5.03 g (3.38 mmol) of a 2.42% hydrogen chloride-toluene solution was added thereto. The mixture was stirred at 90°C for 5 hours. The E isomer/Z isomer ratio of the reaction mixture, when measured by HPLC, was 3.24 : 96.76. The reaction mixture was cooled to room temperature, and crystals were filtered and washed with acetone (20 mL). The crystals were dried under reduced pressure to obtain 1.46 g of (Z)-11-(3-dimethylaminopropylidene)-6,11-dihydrodibenz[b,e] oxepin-2-acetic acid • hydrochloride. The yield was 69.4%. The proportions of the Z isomer and the E isomer, when measured by HPLC, were 99.13% and 0.87%, respectively.
¹H NMR (400 MHz, DMSO-d₆) δ 2.73 (s, 6H), 2.77 (td, J = 7.6, 7.2 Hz, 2H), 3.25 (t, J = 7.6 Hz, 2H), 3.55 (s, 2H), 5.21 (brs, 1H), 5.65 (t, J = 7.2 Hz, 1H), 6.79 (d, J = 8.4 Hz, 1H), 7.07 (d, J = 2.0 Hz, 1H), 7.10 (dd, J = 8.0, 2.0 Hz, 1H), 7.28-7.40 (m, 4H), 10.28 (brs, 1H), 12.31 (brs, 1H)

### Industrial Applicability

The present invention enables the production of olopatadine, namely, (Z)-11-(3-dimethylaminopropylidene)-6, 11-dihydrodibenz[b,e] oxepin-2-acetic acid, which is useful as a medicinal agent, in a high yield and in an industrially advantageous manner.

## Claims

1. A process for producing (2)-11-(3-dimethylaminopropylidene)-6,11-dihydrodibenz[b,e]oxepin-2-acetic acid represented by Formula [II]: 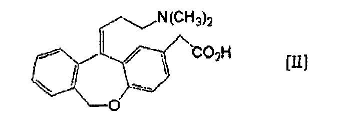 or an acid addition salt thereof, the process comprising subjecting 11-hydroxy-11-(3-dimethylaminopropyl)-6,11-dihydrodibenz[b,e]oxepin-2-acetic acid represented by Formula [I]: 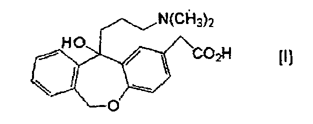 or a salt thereof to a heat treatment in a solvent and a water removal treatment in a reaction system in the presence of an acid selected from a group consisting of hydrogen chloride, sulfuric acid, methanesulfonic acid and p-toluenesulfonic acid.

2. The process according to claim 1, wherein the water removal treatment in the reaction system is performed by using a C₃-C₈ carboxylic acid anhydride.

3. The process according to claim 2, wherein the C₃-C₈ carboxylic acid anhydride is acetic anhydride.

4. The process according to claim 1, wherein the water removal treatment in the reaction system is performed by azeotropic distillation.

5. The process according to claim 4, wherein the azeotropic distillation is performed under reduced pressure.

6. The process according to claim 1, wherein the water removal treatment in the reaction system is performed by using a C₂-C₈ carboxylic halide.

7. The process according to claim 6, wherein the C₂-C₈ carboxylic halide is acetyl chloride.

8. The process according to claim 1, wherein the water removal treatment in the reaction system is performed by using an oxyhalide of sulfur or phosphorus.

9. The process according to claim 1, wherein the acid is hydrogen chloride.

10. The process according to claim 1, wherein the heat treatment is performed at 70 to 120°C.

11. The process according to claim 1, which is performed in the presence of a seed crystal of (Z)-11-(3-dimethylaminopropylidene)-6,11-dihydrodibenz[b,e] oxepin-2-acetic acid or an acid addition salt thereof.

12. The process according to claim 1, wherein the organic solvent is toluene or chlorobenzene.

## Patentansprüche

1. Ein Verfahren zur Herstellung von (Z)-11-(3-Dimethylaminopropyliden)-6,11-dihydrodibenz[b,e]oxepin-2-essigsäure, dargestellt durch Formel [II]: 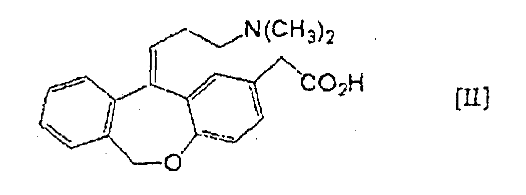 oder eines Säureadditionssalzes davon, wobei das Verfahren umfasst Unterziehen von 11-Hydroxy-11-(3-dimethylaminopropyl)-6,11-dihydrodibenz[b,e]oxepin-2-essigsäure, dargestellt durch Formel [I]: 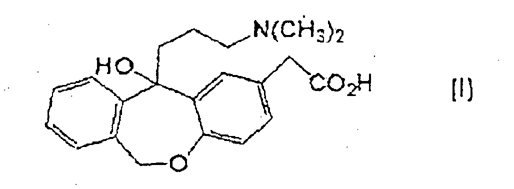 oder eines Salzes davon einer Wärmebehandlung in einem Lösungsmittel und einer Wasserentzugsbehandlung in einem Reaktionssystem in Gegenwart einer Säure, ausgewählt aus einer Gruppe bestehend aus Chlorwasserstoff, Schwefelsäure, Methansulfonsäure und p-Toluolsulfonsäure.

2. Das Verfahren nach Anspruch 1, wobei die Wasserentzugsbehandlung in dem Reaktionssystem unter Verwendung eines C₃-C₈-Carbonsäureanhydrids durchgeführt wird.

3. Das Verfahren nach Anspruch 2, wobei das C₃-C₈-Carbonsäureanhydrid Essigsäureanhydrid ist.

4. Das Verfahren nach Anspruch 1, wobei die Wasserentzugsbehandlung in dem Reaktionssystem durch azeotrope Destillation erfolgt.

5. Das Verfahren nach Anspruch 4, wobei die azeotrope Destillation unter vermindertem Druck erfolgt.

6. Das Verfahren nach Anspruch 1, wobei die Wasserentzugsbehandlung in dem Reaktionssystem unter Verwendung eines C₂-C₈-Carbonsäurehalogenids erfolgt.

7. Das Verfahren nach Anspruch 6, wobei das C₂-C₈-Carbonsäurchalogenid Acetylchlorid ist.

8. Das Verfahren nach Anspruch 1, wobei die Wasserentzugsbehandlung in dem Reaktionssystem unter Verwendung eines Oxyhalogenids von Schwefel oder Phosphor erfolgt.

9. Das Verfahren nach Anspruch 1, wobei die Säure Chlorwasserstoff ist.

10. Das Verfahren nach Anspruch 1, wobei die Wärmebehandlung bei 70 bis 120°C erfolgt.

11. Das Verfahren nach Anspruch 1, welches in Gegenwart eines Impfkristalls von (Z)-11-(3-Dimethylaminopropyliden)-6,11-dihydrobenz[b,e]oxepin-2-essigsäure oder eines Säureadditionssalzes davon durchgeführt wird.

12. Das Verfahren nach Anspruch 1, wobei das organische Lösungsmittel Toluol oder Chlorbenzol ist.

## Revendications

1. Procédé de production d'acide (Z)-11-(3-diméthylaminopropylidène)-6,11-dihydrodibenz[b,e]oxépin-2-acétique représenté par la formule [II] : 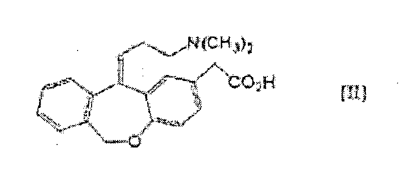 ou un sel d'addition d'acide de celui-ci, le procédé comprenant la soumission d'acide 11-hydroxy-11-(3-diméthylaminopropyl)-6,11-dihydrodibenz[b,e]oxépin-2-acétique représenté par la formule [I] : 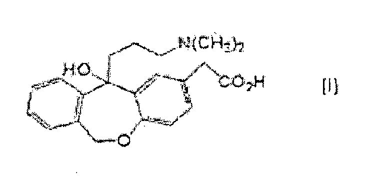 ou un sel de celui-ci à un traitement thermique dans un solvant et un traitement d'élimination d'eau dans un système de réaction en présence d'un acide choisi dans un groupe constitué des chlorure d'hydrogène, acide sulfurique, acide méthanesulfonique et acide p-toluènesulfonique.

2. Procédé selon la revendication 1, dans lequel le traitement d'élimination d'eau dans le système de réaction est effectué en utilisant un anhydride d'acide carboxylique en C₃-C₈.

3. Procédé selon la revendication 2, dans lequel l'anhydride d'acide carboxylique en C₃-C₈ est l'anhydride acétique.

4. Procédé selon la revendication 1, dans lequel le traitement d'élimination d'eau dans le système de réaction est effectué par distillation azéotrope.

5. Procédé selon la revendication 4, dans lequel la distillation azéotrope est effectuée sous pression réduite.

6. Procédé selon la revendication 1, dans lequel le traitement d'élimination d'eau dans le système de réaction est effectué en utilisant un halogénure carboxylique en C₂-C₈.

7. Procédé selon la revendication 6, dans lequel l'halogénure carboxylique en C₂-C₈ est le chlorure d'acétyle.

8. Procédé selon la revendication 1, dans lequel le traitement d'élimination d'eau dans le système de réaction est effectué en utilisant un oxyhalogénure de soufre ou de phosphore.

9. Procédé selon la revendication 1, dans lequel l'acide est le chlorure d'hydrogène.

10. Procédé selon la revendication 1, dans lequel le traitement thermique est effectué à 70 à 120 °C.

11. Procédé selon la revendication 1, qui est conduit en présence d'un germe cristallin d'acide (Z)-11-(3-diméthylaminopropylidène)-6,11-dihydrodibenz[b,e]oxépin-2-acétique ou un sel d'addition d'acide de celui-ci.

12. Procédé selon la revendication 1, dans lequel le solvant organique est le toluène ou le chlorobenzène.
